# EUROPEAN PATENT APPLICATION

(11) **EP 4 775 124 A1**
(43) Date of publication of application: **15.07.2026**
(21) Application number: 25191345.5
(22) Date of filing: 23.07.2025
(51) Int. Cl.: A61B 1/00, A61B 1/307, A61M 27/00, A61M 25/06

(54) **URETERAL ACCESS SHEATH**

(30) Priority: 08.01.2025 CN 202510025353
(71) Applicant: Hunan Ruibang Medical Technology Development Co., Ltd., Zhuzhou, Hunan 412000 (CN)
(72) Inventor: HE, Yifeng, Zhuzhou, 412000 (CN); DENG, Xu, Zhuzhou, 412000 (CN)
(74) Representative: Murgitroyd & Company

(57) **Abstract**

Provided is a ureteral access sheath, including a sheath tube, at least one water inlet pipe, and a heat shrink tubing. The sheath tube is of a hollow structure. The sheath tube and one part of the water inlet pipe are nested inside the heat shrink tubing. The water inlet pipe and the sheath tube form a multi-cavity structure. The water inlet pipe is provided with a first water outflow portion at an end close to a water outlet, while the heat shrink tubing is provided with a second water outflow portion at an end close to the water outlet, and the first water outflow portion and the second water outflow portion overlap and are away from the sheath tube. In this manner, when an end of the ureteral access sheath is bent, the pressure at the end can be eliminated, which is more convenient for operation.

## Description

### TECHNICAL FIELD

The present disclosure relates to the field of medical devices, and in particular, to a ureteral access sheath.

### BACKGROUND

An access sheath, as a medical device commonly used in urinary surgeries, is mainly used in a ureteroscopic calculus removal procedure. In the ureteroscopic calculus removal procedure, the access sheath first enters into the body of a patient, and is directly advanced to be in the vicinity of a lesion location visually so as to assist with an endoscope and other instruments entering a lesioned organ (such as the kidney, the gallbladder, or the ureter), providing a continuous operation channel therefor. The lesioned organ can be protected when the instruments are changed repeatedly such that injuries to the lesioned organ can be reduced. Meanwhile, precision instruments can be protected against damage.

In the ureteroscopic calculus removal procedure, the lesioned organ may sometimes be shriveled and need to be injected with normal saline to become full, and the broken calculus needs to be rinsed and removed. The first-generation access sheath has only one channel that is used for instruments such as an endoscope to enter while allowing a water inlet pipe to pass therethrough to keep a lesioned organ full. Consequently, a doctor needs to repeatedly change the instruments (using the endoscope for observation and using a water inlet device to feed water). In order to facilitate the doctor's operations, several improvement solutions have been available on the market: the first one is to provide an updated instrument that has one or more of endoscopy, calculus removal, and water feed functions, etc.; the second one is to add a water inlet channel to an access sheath (e.g., CN104644242A); and the third one is to provide a dilator at the tail end of an access sheath (e.g., CN219595583U). For the first improvement solution, since the size of the access sheath cannot be too large (because a too large size may cause a large wound to the human body), increasing requirements may be imposed on the instrument with increasing integrated functions, and in the repeated calculus removal process by the doctor, the pressure within the lesioned organ may not be controlled easily, leading to increased operation difficulty. The complex internal environment of the lesioned organ is not taken into account. For example, the kidney has a plurality of chambers, and the ends of the access sheath and other instruments are often required to be bendable. For the second improvement solution, since the internal environment of the lesioned organ is complex, for example, the kidney has a plurality of chambers, the ends of the access sheath and other instruments are often required to be bendable, and if water is injected in the bending process, a pressure will be produced at the position of bending, resulting in difficult operation. For the third improvement solution, since the internal environment of the lesioned organ is complex, for example, the kidney has a plurality of chambers, the dilator cannot be fit with the plurality of chambers and cannot accommodate the complex environment. That is, the exiting improvement solutions do not take into account the case in which the tail end of the access sheath needs to be bent, resulting in difficult operation.

### SUMMARY

In view of the shortcomings of the prior art, an objective of the present disclosure is to provide a ureteral access sheath to reduce the operation difficulty.

In order to achieve the above objective, the present disclosure provides the following technical solution: a ureteral access sheath includes a sheath tube, at least one water inlet pipe, and a heat shrink tubing. The sheath tube is of a hollow structure for an endoscope to pass therethrough. The sheath tube and one part of the water inlet pipe are nested inside the heat shrink tubing. The water inlet pipe and the sheath tube form a multi-cavity structure. The water inlet pipe is provided with a first water outflow portion at an end close to a water outlet, while the heat shrink tubing is provided with a second water outflow portion at an end close to the water outlet, and the first water outflow portion and the second water outflow portion overlap and are away from the sheath tube.

In an embodiment, a cross section of the water inlet pipe includes a linear portion and a curved portion that form a D-shape; and the linear portion is in contact with the sheath tube.

In an embodiment, the first water outflow portion includes at least one water outflow hole, and the second water outflow portion includes an opening for exposing the water outflow hole.

In an embodiment, the at least one water inlet pipe shares a same water inlet, and has water outlets distributed along a circumferential direction of the sheath tube.

In an embodiment, a reinforcement rib is disposed on an inner wall of the water inlet pipe along a length direction of the water inlet pipe.

In an embodiment, each water inlet pipe is provided with a plurality of reinforcement ribs that are circumferentially disposed at intervals along the inner wall of the water inlet pipe, with a first gap being formed between two adjacent reinforcement ribs.

In an embodiment, the reinforcement rib is in a shape of a lug boss, and an upper surface of the reinforcement rib is arc-shaped.

In an embodiment, a length of the reinforcement rib is equal to a length of the water inlet pipe.

In an embodiment, an end of the sheath tube away from the water outlet is communicated with a Y-shaped connector that includes a main channel and a branch channel; the main channel is communicated with the sheath tube to allow an endoscope to pass therethrough; the branch channel has one end communicated with the main channel and the other end communicated with a negative pressure suction assembly; a rubber sealing ring is disposed within the main channel; and a through hole matching the endoscope is formed in the rubber sealing ring.

In an embodiment, the other part of the water inlet pipe is inclined outwardly along an external surface of the sheath tube.

In an embodiment, an angle of inclination between the water inlet pipe and the sheath tube is 20° to 30°.

In an embodiment, an angle fixator is disposed at a position where the water inlet pipe is inclined along the sheath tube.

Compared with the prior art, the above technical solution has the following technical effects.

The ureteral access sheath of the present disclosure includes the sheath tube, at least one water inlet pipe, and the heat shrink tubing. The sheath tube is of the hollow structure. The sheath tube and one part of the water inlet pipe are nested inside the heat shrink tubing. The water inlet pipe and the sheath tube form the multi-cavity structure. The water inlet pipe is provided with the first water outflow portion at the end close to the water outlet, while the heat shrink tubing is provided with the second water outflow portion at the end close to the water outlet, and the first water outflow portion and the second water outflow portion overlap and are away from the sheath tube. In this manner, on the basis of providing the water inlet pipe and forming the multi-cavity structure with the sheath tube, the present disclosure further has the water outflow portion at the tail end of the water inlet pipe so that when an end of the ureteral access sheath is bent, the pressure at the end can be eliminated, which is more convenient for operation.

### BRIEF DESCRIPTION OF THE DRAWINGS

To describe the technical solutions in the embodiments of the present disclosure more clearly, the following briefly describes the accompanying drawings required for describing the embodiments. It should be understood that the following accompanying drawings show merely some embodiments of the present disclosure, and therefore should not be regarded as a limitation on the scope. A person of ordinary skill in the art may still derive other related drawings from these accompanying drawings without creative efforts.
FIG. 1 is a structural schematic diagram of a water outflow end of a ureteral access sheath according to an embodiment of the present disclosure;
FIG. 2 is an enlarged structural schematic diagram of a water outflow end of a ureteral access sheath according to an embodiment of the present disclosure;
FIG. 3 is another enlarged structural schematic diagram of a water outflow end of a ureteral access sheath according to an embodiment of the present disclosure; and
FIG. 4 is a three-dimensional structural schematic diagram of a ureteral access sheath according to an embodiment of the present disclosure.

### List of Reference Numerals:

10, sheath tube; 20, water inlet pipe; 30, heat shrink tubing; 21, first water outflow portion; 31, second water outflow portion; 22, reinforcement rib; 23, first gap; 24, water outlet; 25, water inlet; 40, Y-shaped connector; 41, main channel; 42, branch channel; and 50, angle fixator.

### DETAILED DESCRIPTION OF THE EMBODIMENTS

The embodiments of the present disclosure are described below in detail. Examples of the embodiments are shown in the drawings. The same or similar numerals represent the same or similar elements or elements having the same or similar functions throughout the specification. The embodiments described below with reference to the accompanying drawings are exemplary. These embodiments are merely used to explain the present disclosure, and should not be construed as a limitation to the present disclosure.

In the description of the present disclosure, the terms "central", "longitudinal", "transverse", "long", "wide", "thick", "upper", "lower", "front", "back", "left", "right", "vertical", "horizontal", "top", "bottom", "inner", "outer", "clockwise", "anticlockwise", "axial", "radial" and "circumferential" etc. are used to indicate orientations shown in the accompanying drawings. It should be noted that these terms are merely intended to facilitate a simple description of the present disclosure, rather than to indicate or imply that the mentioned apparatus or elements must have the specific orientation or be constructed and operated in the specific orientation. Therefore, these terms may not be construed as a limitation to the present disclosure.

Moreover, the terms such as "first" and "second" are used only for the purpose of description and should not be construed as indicating or implying a relative importance, or implicitly indicating a quantity of indicated technical features. Thus, features defined with "first" and "second" may explicitly or implicitly include one or more of the features. In the description of the present disclosure, "a plurality of" means two or more, unless otherwise specifically defined.

In the present disclosure, unless otherwise clearly specified, the terms "installation", "interconnection", "connection" and "fixation" etc. are intended to be understood in a broad sense. For example, the "connection" may be a fixed connection, removable connection or integral connection; may be a mechanical connection or electrical connection; may be a direct connection or indirect connection using a medium; and may be a communication or interaction between two elements. Those of ordinary skill in the art may understand specific meanings of the above terms in the present disclosure based on a specific situation.

In the present disclosure, unless otherwise clearly specified and limited, when it is described that a first feature is "above" or "below" a second feature, it indicates that the first and second features are in direct contact or the first and second features are in indirect contact through a medium. In addition, when it is described that the first feature is "over", "above" and "on" the second feature, it indicates that the first feature is directly or obliquely above the second feature, or simply indicates that an altitude of the first feature is higher than that of the second feature. When it is described that the first feature is "under", "below" or "beneath" the second feature, it indicates that the first feature is directly or obliquely under the second feature or simply indicates that an altitude of the first feature is lower than that of the second feature.

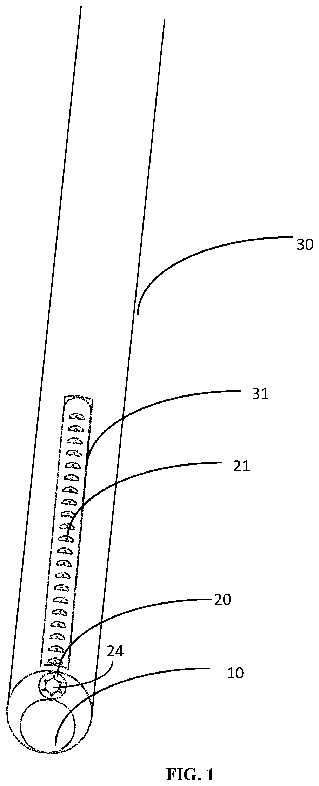

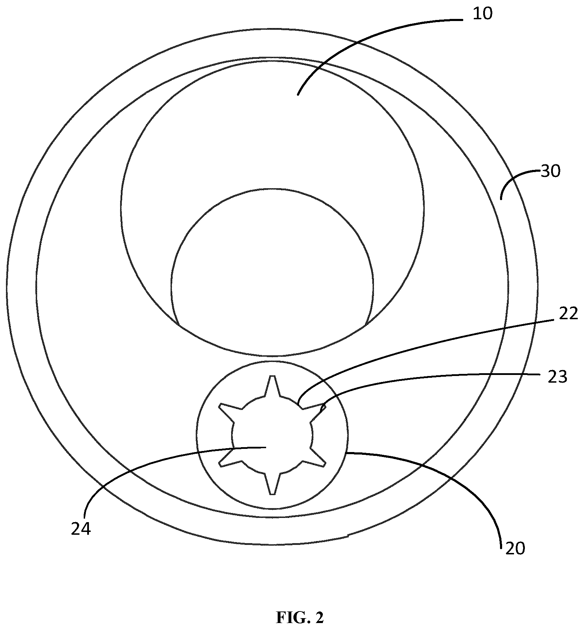

As shown in FIG. 1 and FIG. 2, the ureteral access sheath of the present embodiment includes a sheath tube 10, at least one water inlet pipe 20, and a heat shrink tubing 30. The sheath tube 10 is of a hollow structure (moreover, a spring ring (not shown) may be disposed within the sheath tube 10 and configured to support the sheath tube 10). The sheath tube 10 is configured for machines such as an endoscope to pass therethrough. At least one water inlet pipe 20 is disposed around the sheath tube 10. The water inlet pipe 20 and the sheath tube 10 form a multi-cavity structure. The sheath tube 10 and one part of the water inlet pipe 20 are nested inside the heat shrink tubing 30. That is, the water inlet pipe 20 has one part nested inside the heat shrink tubing 30 and the other part exposed. The water inlet pipe 20 is provided with a first water outflow portion 21 at an end close to a water outlet 24, while the heat shrink tubing 30 is provided with a second water outflow portion 31 at an end close to the water outlet 24, and the first water outflow portion 21 and the second water outflow portion 31 overlap and are away from the sheath tube 10. That is, a water outflow direction of the first water outflow portion 21 does not point to the sheath tube 10, thereby facilitating liquid discharge. As shown in FIG. 1, the first water outflow portion 21 includes at least one water outflow hole; and the water outflow hole and the second water outflow portion 31 are arranged along a length direction of the sheath tube 10. When the tail end of the ureteral access sheath is straight, most of the liquid in the water inlet pipe 20 flows out of the water outlet 24, while a small amount of the liquid flows out of the first water outflow portion 21. When the tail end of the ureteral access sheath is bent, most of the liquid flows out of the water outflow portion 21, while a small amount of the liquid flows out of the water outlet 24. Thus, the ureteral access sheath can be bent arbitrarily and can be better adapted to various use scenarios. The use difficulty is reduced. At present, after testing, a similar surgical procedure in which the doctor uses the existing access sheath typically takes more than 3 hours, and more than half of the time can be reduced when the ureteral access sheath of the present disclosure is used.

The ureteral access sheath of the present disclosure includes the sheath tube, at least one water inlet pipe, and the heat shrink tubing. The sheath tube is of the hollow structure. The sheath tube and one part of the water inlet pipe are nested inside the heat shrink tubing. The water inlet pipe and the sheath tube form the multi-cavity structure. The water inlet pipe is provided with the first water outflow portion at the end close to the water outlet, while the heat shrink tubing is provided with the second water outflow portion at the end close to the water outlet, and the first water outflow portion and the second water outflow portion overlap and are away from the sheath tube. In this manner, on the basis of providing the water inlet pipe and forming the multi-cavity structure with the sheath tube, the present disclosure further has the water outflow portion at the tail end of the water inlet pipe so that when an end of the ureteral access sheath is bent, the pressure at the end can be eliminated, which is more convenient for operation.

In an embodiment, in order to better form a hole in the first water outflow portion, an opening may be first formed in an end of the heat shrink tubing 30 close to a water outlet of the ureteral access sheath, and then a hole is formed in an end of the water inlet pipe 20 close to the water outlet of the ureteral access sheath after heat shrinkage. Thus, the first water outflow portion 21 includes at least one water outflow hole, and the second water outflow portion 31 includes the opening for exposing the water outflow hole. In particular implementation, the opening may not be first formed in the end of the heat shrink tubing 30 close to the water outlet of the ureteral access sheath, and holes are formed in ends of the water inlet pipe 20 and the heat shrink tubing 30 close to the water outlet of the ureteral access sheath after heat shrinkage.

In an embodiment, the at least one water inlet pipe 20 shares a same water inlet 25, and has water outlets distributed along a circumferential direction of the sheath tube 10. Thus, the at least one water inlet pipe 20 is distributed in the circumferential direction of the sheath tube 10, and each water inlet pipe 20 is provided with the water outlet. The water outflow effect can be guaranteed regardless of the ureteral access sheath being bent at any angle.

In an embodiment, with continued reference to FIG. 1 and FIG. 2, a reinforcement rib 22 is disposed on an inner wall of the water inlet pipe 20 along a length direction of the water inlet pipe 20. When bending, the reinforcement rib 22 first comes into contact with the wall of the water inlet pipe 20, and the other part still keeps unobstructed. Therefore, with the reinforcement rib 22 disposed within the water inlet pipe 20, the blockage of the water inlet pipe caused by bending can be avoided. In an embodiment, if there is one reinforcement rib 22, the optimal embodiment is that the reinforcement rib 22 is located in the plane where a center of circle of the sheath tube 10 and a center of circle of the water inlet pipe 20 are positioned (the center of circle of the sheath tube 10, the center of circle of the water inlet pipe 20, and a midpoint of a cross section of the reinforcement rib 22 are located in the same straight line). In this case, since a diameter of the sheath tube 10 is greater than that of the water inlet pipe 20, no matter how the sheath tube 10 is bent, the water inlet pipe 20 will not be flattened (if the sheath tube 10 is bent along a first direction that is perpendicular to the straight line in which the center of circle of the sheath tube 10, the center of circle of the water inlet pipe 20, and the reinforcement rib 22 are positioned, since the water inlet pipe 20 will slide in the heat shrink tubing 30 when bending, a bending degree of the water inlet pipe 20 will be smaller than that of the sheath tube 10). Thus, it can be guaranteed to the utmost extent that the passage of the water inlet pipe 20 is not blocked.

In an embodiment, with continued reference to FIG. 2, each water inlet pipe 20 is provided with a plurality of reinforcement ribs 22 that are circumferentially disposed at intervals along the inner wall of the water inlet pipe 20, with a first gap 23 being formed between two adjacent reinforcement ribs 22. The first gap 23 is provided mainly for guaranteeing that the water inlet pipe 20 can be bent without affecting use in the bent state.

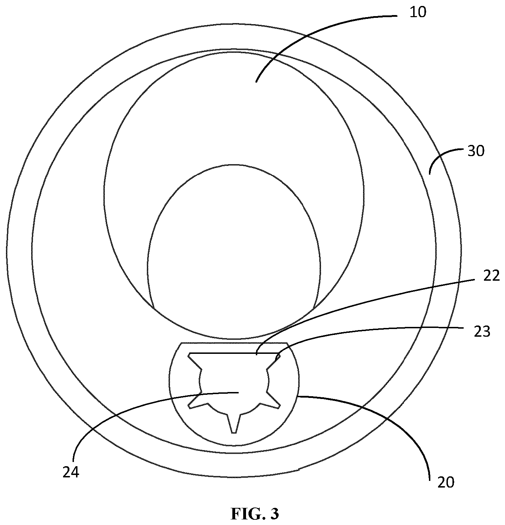

In an embodiment, with continued reference to FIG. 2, the reinforcement rib 22 is in the shape of a lug boss, and an upper surface of the reinforcement rib 22 is arc-shaped. The cross section of the reinforcement rib is approximately trapezoidal with the difference that the upper surface is arc-shaped. When the plurality of reinforcement ribs 22 are bent, the plurality of reinforcement ribs 22 approach each other, and a passage may be formed in the middle, thereby guaranteeing water feed. In particular implementation, the reinforcement rib 22 may also be in other shapes. For example, the cross section of the reinforcement rib is rectangular, square, etc. In the present disclosure, the water inlet pipe 20 may be circular, and of course, may also be in other shapes. As shown in FIG. 3, in another embodiment, the water inlet pipe 20 is D-shaped. The water inlet pipe 20 includes a linear portion in combination with a curved portion. The linear portion and the curved portion are connected end to end on both sides. Thus, the shape of the cross section is D-shape. The linear portion is in contact with the sheath tube 10. If the overall size of the ureteral access sheath is unchanged, the water inlet pipe 20 is D-shaped, and compared with a circular water inlet pipe, the diameter of the sheath tube 10 may be relatively larger, which is more convenient for a doctor to conduct surgical operations. If the diameter of the sheath tube 10 is identical, the D-shaped water inlet pipe 20 has a smaller cross section than the circular water inlet pipe 20 such that the overall size of the ureteral access sheath can be made smaller. In this way, the wound to the human body may be smaller, which is more conducive to healing.

In an embodiment, a length of the reinforcement rib 22 is equal to that of the water inlet pipe 20. In particular implementation, generally, only the water outlet part of the ureteral access sheath will be bent in the normal use process. Hence, the reinforcement rib 22 may be disposed merely at one part of the water inlet pipe 20 close to the water outlet. The length of the reinforcement rib 22 is equal to that of the water inlet pipe 20 so that unforeseen circumstances caused by bending of the other part due to improper use for other reasons can be avoided.

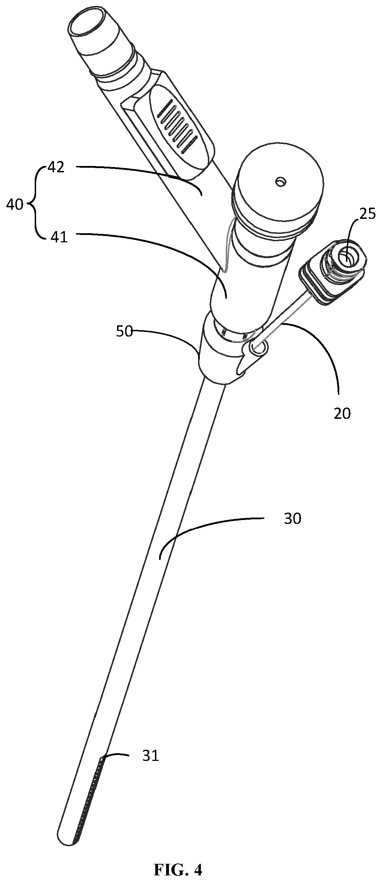

In an embodiment, with reference to FIG. 4, an end of the sheath tube 10 away from the water outlet of the ureteral access sheath is communicated with a Y-shaped connector 40 that includes a main channel 41 and a branch channel 42. The main channel 41 is communicated with the sheath tube 10 to allow instruments such as an endoscope to pass therethrough. The branch channel 42 has one end communicated with the main channel 41 and the other end communicated with a negative pressure suction assembly (not shown). A rubber sealing ring (not shown) is disposed within the main channel 41. A through hole matching the endoscope is formed in the rubber sealing ring. A calculus removal instrument goes into the sheath tube 10 from the main channel 41. After the calculus is taken out, the negative pressure suction assembly draws the liquid and the calculus out of the branch channel 42. The through hole matching the endoscope is formed in the rubber sealing ring, thereby preventing leaking liquid and air from entering the branch channel 42 from the main channel 41 and avoiding influence on the negative pressure suction assembly drawing the liquid and the calculus out of the branch channel 42.

In an embodiment, with reference to FIG. 4, in order to facilitate communication of the water inlet pipe 20 with an external liquid, the other part of the water inlet pipe 20 is inclined outwardly along an external surface of the sheath tube 10. In a particular embodiment, an angle of inclination between the water inlet pipe 20 and the sheath tube 10 is 20° to 30°. Thus, it can be guaranteed that the water inlet pipe 20 will not be bent and flattened, guaranteeing the water feeding effect, and the communication of the water inlet pipe 20 with the external liquid may also be facilitated.

In an embodiment, with reference to FIG. 4, since the water inlet pipe 20 is a flexible pipe, in order to guarantee the angle of inclination of the water inlet pipe 20, an angle fixator 50 is disposed at a position where the water inlet pipe 20 is inclined along the sheath tube 10. A main body of the angle fixator 50 is sleeved on the heat shrink tubing 30. The water inlet pipe 20 passes through an auxiliary tube of the angle fixator 50, thereby limiting the moving space of the water inlet pipe 20 and guaranteeing the angle of inclination between the water inlet pipe 20 and the sheath tube 10.

In this specification, descriptions of reference terms such as "one embodiment", "some embodiments", "an example", "a specific example", and "some examples" indicate that specific features, structures, materials, or characteristics described in combination with the embodiment(s) or example(s) are included in at least one embodiment or example of the present disclosure. In this specification, the schematic expression of the above terms is not necessarily directed to the same embodiment or example. Moreover, the specific features, structures, materials, or characteristics described may be combined in a suitable manner in any one or more embodiments or examples. In addition, those skilled in the art may combine different embodiments or examples described in this specification and characteristics of the different embodiments or examples without mutual contradiction.

Although the embodiments of the present disclosure have been shown and described above, it can be understood that the foregoing embodiments are exemplary, and cannot be construed as limitations to the present disclosure. A person of ordinary skill in the art may make changes, modifications, replacements, and variations to the foregoing embodiments. These changes, modifications, replacements, and variations (e.g., different combinations of specific embodiments, or different combinations of distinguishing technical features) shall all fall within the protection scope claimed in the present disclosure as long as they conform to the objective of the present disclosure.

## Claims

1. A ureteral access sheath, comprising: a sheath tube (10), at least one water inlet pipe (20), and a heat shrink tubing (30), wherein the sheath tube (10) is of a hollow structure; the sheath tube (10) and one part of the water inlet pipe (20) are nested inside the heat shrink tubing (30); the water inlet pipe (20) and the sheath tube (10) form a multi-cavity structure; the water inlet pipe (20) is provided with a first water outflow portion (21) at an end close to a water outlet of the water inlet pipe (20), while the heat shrink tubing (30) is provided with a second water outflow portion (31) at an end close to the water outlet, and the first water outflow portion (21) and the second water outflow portion (31) overlap and are away from the sheath tube (10); the first water outflow portion (21) comprises at least one water outflow hole; and the water outflow hole and the second water outflow portion (31) are arranged along a length direction of the sheath tube (10).

2. The ureteral access sheath according to claim 1, wherein a cross section of the water inlet pipe (20) comprises a linear portion and a curved portion that form a D-shape; and the linear portion is in contact with the sheath tube (10).

3. The ureteral access sheath according to claim 1, wherein the second water outflow portion (31) comprises an opening for exposing the water outflow hole.

4. The ureteral access sheath according to claim 1, wherein the at least one water inlet pipe (20) shares a same water inlet, and has water outlets distributed along a circumferential direction of the sheath tube (10).

5. The ureteral access sheath according to claim 1, wherein a reinforcement rib (22) is disposed on an inner wall of the water inlet pipe (20) along a length direction of the water inlet pipe (20).

6. The ureteral access sheath according to claim 5, wherein each water inlet pipe (20) is provided with a plurality of reinforcement ribs (22) that are circumferentially disposed at intervals along the inner wall of the water inlet pipe (20), with a first gap (23) being formed between two adjacent reinforcement ribs (22).

7. The ureteral access sheath according to claim 5 or 6, wherein the reinforcement rib (22) is in a shape of a lug boss, and an upper surface of the reinforcement rib (22) is arc-shaped.

8. The ureteral access sheath according to claim 5, wherein a length of the reinforcement rib (22) is equal to a length of the water inlet pipe (20).

9. The ureteral access sheath according to claim 1, wherein an end of the sheath tube (10) away from the water outlet is communicated with a Y-shaped connector (40), the Y-shaped connector (40) comprises a main channel (41) and a branch channel (42); the main channel (41) is communicated with the sheath tube (10) to allow an endoscope to pass therethrough; the branch channel (42) has one end communicated with the main channel (41) and the other end communicated with a negative pressure suction assembly; a rubber sealing ring is disposed within the main channel (41); and a through hole matching the endoscope is formed in the rubber sealing ring.

10. The ureteral access sheath according to claim 1, wherein the other part of the water inlet pipe (20) is inclined outwardly along an external surface of the sheath tube (10).

## Amended claims

### Amended claims in accordance with Rule 137(2) EPC.

1. A ureteral access sheath, comprising: a sheath tube (10), at least one water inlet pipe (20), and a heat shrink tubing (30), wherein the sheath tube (10) is of a hollow structure; the sheath tube (10) and one part of the water inlet pipe (20) are nested inside the heat shrink tubing (30); the water inlet pipe (20) and the sheath tube (10) form a multi-cavity structure; the water inlet pipe (20) is provided with a first water outflow portion (21) at an end close to a water outlet of the water inlet pipe (20), while the heat shrink tubing (30) is provided with a second water outflow portion (31) at an end close to the water outlet, and the first water outflow portion (21) and the second water outflow portion (31) overlap and are away from the sheath tube (10); the first water outflow portion (21) comprises at least one water outflow hole; and the water outflow hole and the second water outflow portion (31) are arranged along a length direction of the sheath tube (10).

2. The ureteral access sheath according to claim 1, wherein a cross section of the water inlet pipe (20) comprises a linear portion and a curved portion that form a D-shape; and the linear portion is in contact with the sheath tube (10).

3. The ureteral access sheath according to claim 1, wherein the second water outflow portion (31) comprises an opening for exposing the water outflow hole.

4. The ureteral access sheath according to claim 1, wherein the at least one water inlet pipe (20) shares a same water inlet, and has water outlets distributed along a circumferential direction of the sheath tube (10).

5. The ureteral access sheath according to claim 1, wherein a reinforcement rib (22) is disposed on an inner wall of the water inlet pipe (20) along a length direction of the water inlet pipe (20).

6. The ureteral access sheath according to claim 5, wherein each water inlet pipe (20) is provided with a plurality of reinforcement ribs (22) that are circumferentially disposed at intervals along the inner wall of the water inlet pipe (20), with a first gap (23) being formed between two adjacent reinforcement ribs (22).

7. The ureteral access sheath according to claim 5 or 6, wherein the reinforcement rib (22) is in a shape of a lug boss, and an upper surface of the reinforcement rib (22) is arc-shaped.

8. The ureteral access sheath according to claim 5, wherein a length of the reinforcement rib (22) is equal to a length of the water inlet pipe (20).

9. The ureteral access sheath according to claim 1, wherein an end of the sheath tube (10) away from the water outlet is communicated with a Y-shaped connector (40), the Y-shaped connector (40) comprises a main channel (41) and a branch channel (42); the main channel (41) is communicated with the sheath tube (10) to allow an endoscope to pass therethrough; the branch channel (42) has one end communicated with the main channel (41) and the other end communicated with a negative pressure suction assembly; a rubber sealing ring is disposed within the main channel (41); and a through hole matching the endoscope is formed in the rubber sealing ring.

10. The ureteral access sheath according to claim 1, wherein another part of the water inlet pipe (20) is inclined outwardly along an external surface of the sheath tube (10).
